# EUROPEAN PATENT APPLICATION

(11) **EP 4 631 542 A1**
(43) Date of publication of application: **15.10.2025**
(21) Application number: 24223285.8
(22) Date of filing: 26.12.2024
(51) Int. Cl.: A61L 31/04, A61L 31/06, A61L 31/14

(54) **BONE AUGMENTATION BAG AND MANUFACTURING METHOD THEREFOR**

(30) Priority: 11.04.2024 WO PCT/JP2024/014678
(71) Applicant: Nihonbashi Implant Center Co., Ltd., Chuo-ku, Tokyo 103-0013 (JP)
(72) Inventor: TAMAKI, Hitoshi, Chuo-ku, Tokyo, 1030013 (JP); MORIMOTO, Shuji, Osaka-shi, Osaka, 5550012 (JP); MICHIWAKI, Takanao, Osaka-shi, Osaka, 5550012 (JP)
(74) Representative: Hasegawa, Kan

(57) **Abstract**

A bag 1 is used for bone augmentation, includes a first film 2a and a second film 2b each made of a biodegradable component, and has an opening 3 between the first film 2a and the second film 2b superimposed and bonded with each other. An in vivo degradation time of the first film 2a is equal to or higher than an in vivo degradation time of the second film 2b, and if the in vivo degradation times of the first film 2a and the second film 2b are different from each other, the first film 2a and the second film 2b are different from each other so as to be visually or tactilely identifiable.

## Description

### TECHNICAL FIELD

The present invention relates to a bag used for bone augmentation and a manufacturing method therefor.

### BACKGROUND ART

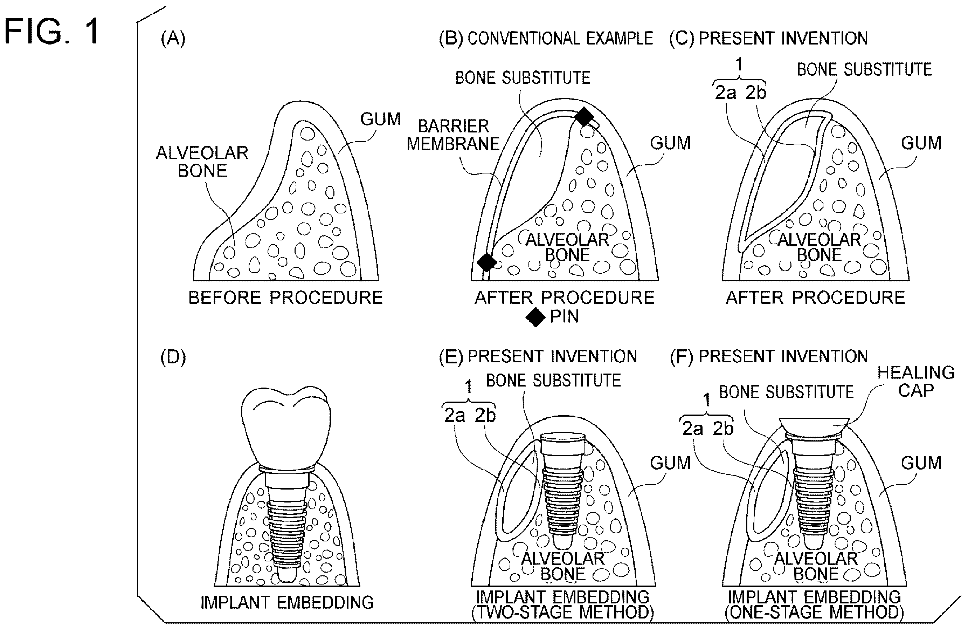

In many cases, bone is lacking when performing implant treatment (see FIGS. 1(A) to (D)). In such cases, in addition to filling with a bone substitute, bone augmentation (bone grafting) is performed using a non-resorbable barrier membrane or a resorbable barrier membrane. When a tooth is extracted, the alveolar bone around the tooth is resorbed, so that the frequency of use of a resorbable barrier membrane is very high.

Existing resorbable barrier membranes (resorbable membranes) are used in the form of a single sheet obtained by solidifying a certain resorbable component (poly-L-lactic acid/poly-D-lactic acid. See: Tadaharu Kobayashi et al., "Clinical Evaluation of Resorbable Poly (L-Lactide/D-Lactide/Glycolide) Bone Fixation Devices in Orthognathic Surgery", The Japanese Journal of Craniofacial Deformities, 2011, Vol. 21, Issue 4, pp. 238-243; Hiroyuki Kano et al., "Postoperative Skeletal Stability after Bimaxillary Orthognathic Surgery in Patients with Mandibular Prognathism using Resorbable Poly (L-Lactide/D-Lactide/Glycolide) Bone Fixations Devices", The Japanese Journal of Craniofacial Deformities, 2013, Vol. 23, Issue 1, pp. 8-14; and Yukihiko Kinoshita et al., "Application of implants to reconstructed jawbone by grafting with resorbable biomaterial poly-L-lactic acid and autologous bone marrow cancellous bone fragment", Head and Neck Cancer, 2000, Vol. 26, No. 3, p. 525-530), and are offered by various companies. If a barrier membrane is not stable, the amount of bone augmentation will decrease, and thus, in order to successfully complete bone augmentation, the barrier membrane has to be stabilized such that the barrier membrane does not move. There is also a group that recommends the use of barrier membrane fixation pins to stabilize a barrier membrane.

The following surgical techniques currently exist as surgical techniques for bone grafting in the guided bone regeneration method (GBR method) after gum incision.
(I) A surgical technique in which a bone substitute is placed as a bone augmentation material at a treatment site in a mouth, and the mucosa is sutured to complete the procedure (hereinafter referred to as "Technique I", in which only a bone substitute is used).
(II) A surgical technique in which a bone substitute is placed as a bone augmentation material at a treatment site in a mouth, a resorbable barrier membrane is placed thereon, and the mucosa is sutured to complete the procedure (hereinafter referred to as "Technique II", in which two types, bone substitute and barrier membrane, are used).
(III) A surgical technique in which, after a barrier membrane is placed as in Technique II, a placed bone substitute and the barrier membrane are fixed using a suture such that the bone substitute and the barrier membrane do not move, and the mucosa is sutured to complete the procedure (hereinafter referred to as "Technique III", in which three types, bone substitute, barrier membrane, and suture, are used).
(IV) A surgical technique in which, in order to solve the problem of Technique II, a barrier membrane is fixed using pins instead of the suture in Technique III, and the mucosa is sutured to complete the procedure (hereinafter referred to as "Technique IV", in which three types, bone substitute, barrier membrane, and pins, are used. See FIG. 1(B)).
(V) A surgical technique in which, in order to solve the problem of Technique II, a titanium frame or a non-resorbable membrane is placed instead of the barrier membrane and fixed using pins such that a placed bone substitute does not move, and the mucosa is sutured to complete the procedure (hereinafter referred to as "Technique V", in which three types, bone substitute, titanium frame (or non-resorbable membrane), and pins, are used).

### SUMMARY OF THE INVENTION

The following three conditions are required for bone regeneration: cells, growth factors, and bases (Shuichi Sato, "Current Status of Periodontal Treatment Using Regenerative Treatment," Nihon University Dental Journal, 2015, Vol. 89, pp. 93-99). Here, examples of bases include bone substitutes, and if a bone substitute is merely placed as in Technique I, the placed bone substitute moves from the placement site, and the amount of bone augmentation is halved (reduced), so that a bone regeneration effect cannot be expected. Therefore, by preventing the bone substitute from moving, a further bone regeneration effect can be expected. For this reason, a resorbable barrier membrane is used as in Techniques II to IV described above, or a titanium frame, which is a non-resorbable material, a non-resorbable membrane, or the like is used as in Technique V.

However, in Technique II, the placed bone substitute moves together with the barrier membrane, and the amount of bone augmentation of the placed bone substitute is halved in many cases. In addition, in Techniques III to V, the intraoral operation required to fix the bone substitute is difficult and complicated, and surgery takes a long time. That is, these surgical techniques are not user-friendly. Currently available resorbable barrier membranes are single sheet-like products.

The invention according to the present disclosure has been made in view of the above circumstances, and an object of the present disclosure is to provide a novel bag and a manufacturing method therefor that can realize user-friendly bone augmentation work.

A bag of the present disclosure is a bag used for bone augmentation, the bag including
a first film and a second film each made of a biodegradable component, wherein
the bag has an opening between the first film and the second film superimposed and bonded with each other,
an in vivo degradation time of the first film is equal to or longer than an in vivo degradation time of the second film, and
if the in vivo degradation times of the first film and the second film are different from each other, the first film and the second film are different from each other so as to be visually or tactilely identifiable.

In the bag of the present disclosure, the biodegradable component may include collagen.

In the bag of the present disclosure, the biodegradable component may include a lactic acid (L)-glycolic acid (G) copolymer.

In the bag of the present disclosure,
an UG ratio of each of the first film and the second film may be in a range of 45/55 to 88/12, and
a proportion of lactic acid in the UG ratio of the first film may be equal to or higher than a proportion of lactic acid in the UG ratio of the second film.

In the bag of the present disclosure, the UG ratio of the first film can be in a range of 55/45 to 88/12.

In the bag of the present disclosure, the UG ratio of the second film can be in a range of 45/55 to 55/45.

In the bag of the present disclosure, an inherent viscosity of the lactic acid-glycolic acid copolymer can be in a range of 0.6 dL/g to 1.4 dL/g.

The bag of the present disclosure can contain a bone substitute.

A manufacturing method for the bag of the present disclosure is a method for manufacturing the bag of the present disclosure, the method including
forming each of the first film and the second film by spreading a material containing the biodegradable component over a flat surface and then drying the material.

A bag of the present disclosure is a bag used for bone augmentation, the bag including
a first film and a second film each made of a biodegradable component, wherein
edge portions of the first film and the second film are bonded with each other,
an in vivo degradation time of the first film is equal to or longer than an in vivo degradation time of the second film, and
if the in vivo degradation times of the first film and the second film are different from each other, the first film and the second film are different from each other so as to be visually or tactilely identifiable.

According to the present disclosure, it is possible to provide a novel bag and a manufacturing method therefor that can realize user-friendly bone augmentation work.

### BRIEF DESCRIPTION OF THE DRAWINGS

FIG. 1 shows examples of: a state where an alveolar bone is chipped before implant treatment (A); a conventional resorbable barrier membrane (B); a bag according to an embodiment of the present disclosure (C); an implant and an artificial tooth (D); and the bag and an implant according to the embodiment of the present disclosure (E) (F).
FIG. 2 shows examples of the shape of the bag according to the embodiment of the present disclosure.
FIG. 3 shows top views each showing an opening of the bag according to the embodiment of the present disclosure.
FIG. 4 shows an example of the shape of another bag according to an embodiment of the present disclosure and examples of the case where an opening is made by a surgeon

### DETAILED DESCRIPTION

Hereinafter, exemplary embodiments of the present disclosure will be described with reference to the drawings, but the invention according to the present disclosure is not limited to these embodiments.

### [Bag 1]

FIG. 1(C) shows a bag 1 according to an exemplary embodiment of the present disclosure. The bag 1 includes a first film 2a and a second film 2b each made of a biodegradable component and used for bone augmentation (bone grafting), and the in vivo degradation time of the first film 2a is equal to or longer than the in vivo degradation time of the second film 2b. That is, the degradation time of the outer first film 2a is the same as or slower (longer) than that of the second film 2b, preferably 4 to 9 months, and more preferably 4 to 6 months. On the other hand, the degradation time of the inner second film 2b is the same as or faster (shorter) than that of the first film 2a, preferably 3 to 6 months, and more preferably 1 to 1.5 months.

The bag 1 is scheduled to be resorbed in the submucosa in about 3 to 9 months. In addition, in a procedure for bone formation using the bag 1, a bone substitute can be held in the bag 1, so that a situation in which the placed bone substitute moves and the amount of bone augmentation of the placed bone substitute is halved can be prevented unlike Techniques I and II described above. In addition, in a procedure using the bag 1, it is sufficient to place the bag 1 containing the bone substitute at a treatment site (a missing part of an alveolar bone) after gum incision, and suture the mucosa. Unlike Techniques III to V, there is no need to perform an operation for fixing the bone substitute to the alveolar bone in the mouth, and there is little concern about damaging the alveolar bone, etc., so that such a procedure is friendly to users (including a surgeon and a patient). Furthermore, the procedure is quicker and cheaper than Techniques III to V.

### (First film 2a, second film 2b)

The first film 2a and the second film 2b constitute a main body of the bag 1 and are components for containing and holding the bone substitute. Examples of the biodegradable component which is the material of the first film 2a and the second film 2b include bovine collagen, atelocollagen, tendon collagen, porcine collagen, and placenta membranes, which have been conventionally used as resorbable membranes. The biodegradable component forming the first film 2a and the second film 2b preferably includes a lactic acid (L)-glycolic acid (G) copolymer (hereinafter also referred to as PLGA). PLGA is characterized by its biodegradability, and when embedded in a body, PLGA is degraded into lactic acid and glycolic acid by hydrolysis, and these are metabolized in the body and eliminated harmlessly as water and carbon dioxide. PLGA is degraded more quickly than polylactic acid (PLA), etc. If a difference in bioresorbable performance is provided between the first film 2a and the second film 2b, it is preferable that the first film 2a having a longer in vivo degradation time is placed closer to the mucosal surface (on the mucosal side) and the second film 2b having a shorter in vivo degradation time is placed closer to the alveolar bone (on the bone side) (see FIG. 1(C)). This is because, since bone regeneration is started from the bone side, the need to fix the bone substitute contained in the bag 1 is higher on the mucosal side than on the bone side, and the mucosal side portion (first film 2a) of the bag 1 is desired to exist for a long time.

As shown in examples (experimental examples), the in vivo degradation rate of PLGA can vary greatly depending on an UG ratio (the ratio of lactic acid (L) to glycolic acid (G). L:G). The degradation rate of PLGA having an UG ratio of 50/50 is considered to be the highest, but in general, the higher the proportion of glycolic acid (G) in PLGA is (in other words, the lower the proportion of L is), the more quickly the PLGA is degraded. This is thought to be because polyglycolic acid (PGA) is more sensitive to water than polylactic acid (PLA) and therefore is degraded more quickly. Therefore, on the assumption that the UG ratio of each of the first film 2a and the second film 2b is in the range of 45/55 to 88/12, the proportion of lactic acid in the UG ratio of the first film 2a which is placed on the mucosal side (the value of L in L:G which is represented such that L + G in PLGA is 100) is preferably equal to or higher than the proportion of lactic acid in the UG ratio of the second film 2b which is placed on the bone side.

If a difference in bioresorbable performance is provided between the first film 2a and the second film 2b, the L/G ratio of the first film 2a is preferably 55/45 to 88/12. Specifically, the UG ratio of the first film 2a can also be in the range of 55/45 to 72/28, 55/45 to 75/25, 55/45 to 82/18, 55/45 to 85/15, 72/28 to 88/12, 78/22 to 88/12, 75/25 to 88/12, 82/18 to 88/12, or 85/15 to 88/12. Meanwhile, if a difference in bioresorbable performance is provided between the first film 2a and the second film 2b, the UG ratio of the second film 2b is preferably in the range of 45/55 to 55/45. Specifically, the UG ratio of the second film 2b can also be in the range of 45/55 to 50/50 or 50/50 to 55/45.

In addition, if no difference in bioresorbable performance is provided between the first film 2a and the second film 2b, the UG ratio of each of the first film 2a and the second film 2b can be in the range of 45/55 to 88/12. Therefore, the UG ratio of the first film 2a may be set in the range of 45/55 to 55/45 or 50/50 to 88/12, and the L/G ratio of the second film 2b may be set in the range of 55/45 to 88/12. As described above, the L/G ratio of each of the first film 2a and the second film 2b may be 45/55, 50/50, 55/45, 72/28, 78/22, 75/25, 82/18, 85/15, or 88/12, and can be designed and changed as appropriate according to the application of the bag 1.

The UG ratio of PLGA significantly affects the degradability (in vivo degradation time and bioresorbable performance), the mechanical properties, and the biocompatibility of the bag 1 and is also considered to be an important factor for expanding the range of the application of the bag 1. The UG ratio can be measured by ¹H-NMR spectroscopy in accordance with the United States Pharmacopeia (USP<761>), for example.

In addition, the inherent viscosity (IV) value of PLGA is preferably 0.6 dL/g or higher and 1.4 dL/g or lower in consideration of the required strength of the bag 1 and appropriate hardness of the bag 1 in the mouth. The IV value can be measured using an Ubbelohde viscometer in accordance with the United States Pharmacopeia (USP<911>), for example.

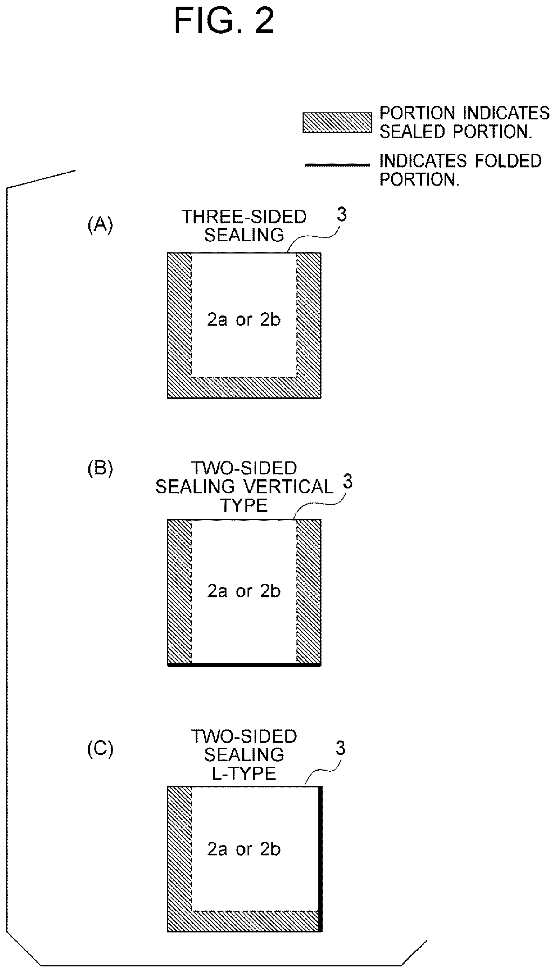

It is preferable that the bag 1 can be folded and is easy to shape. In addition, the size (volume) and the shape of the bag 1 and the dimensions (thickness, length, etc.) of the first film 2a and the second film 2b are not particularly limited, and the bag 1, the first film 2a, and the second film 2b may be formed in a quadrangular shape (FIG. 2), a disk, a trapezoid, a triangular shape, etc. When containing the bone substitute or during the procedure, the bag 1 may be trimmed to match the actual teeth or the row of teeth. In consideration of margins for this, for example, if the bag 1 has a quadrangular shape, the length of one side of the bag 1 can be 13 mm to 17 mm.

If the in vivo degradation times of the first film 2a and the second film 2b are the same, there is no need to distinguish the first film 2a and the second film 2b from each other, and it makes no difference which surface of the bag 1 is placed at the treatment site (bone side, mucosal side).

If the in vivo degradation times of the first film 2a and the second film 2b are different from each other (if a difference in bioresorbable performance is provided therebetween), the first film 2a and the second film 2b are made different from each other such that the first film 2a and the second film 2b are visually or tactilely identifiable by the user of the bag 1 (e.g., the surgeon performing implant treatment). Thus, the surgeon using the bag 1 for the procedure can easily grasp the positions of the first film 2a and the second film 2b in the bag 1 (i.e., the front and back of the bag 1), and can place the desired surface of the bag 1 (the first film 2a or the second film 2b, or the desired position on these films) at the treatment site (e.g., the bone side).

Examples of the manner in which the first film 2a and the second film 2b are "visually identifiable" include marking on one of the first film 2a and the second film 2b and different markings on both of the first film 2a and the second film 2b. Here, the term "marking" refers to the application of marks such as letters, numbers, symbols, lines, and patterns, and these marks are also not particularly limited. For example, the provision of a cell (blank) for the surgeon to mark by themselves is also included in the "visually identifiable manner". These marking methods are also not particularly limited, and examples thereof include handwriting, engraving, stamping, labeling, heat treatment, etc. In addition, the first film 2a and the second film 2b may be made "visually different" using coloring agents, dyes, etc., as in the examples described later.

The manner in which the first film 2a and the second film 2b are "tactilely identifiable" is also not particularly limited, and the first film 2a and/or the second film 2b may be subjected to embossing, in which recesses and projections are provided (Braille characters, patterns, or the like may be provided), surface treatment such as heat treatment, foaming treatment, or the like such that the texture of each film is different.

Furthermore, as an example of the manner in which the first film 2a and the second film 2b are "visually or tactilely identifiable", the first film 2a and the second film 2b may be processed into different shapes (appearances). For example, holes or slits through which the contained bone substitute does not leak may be provided in the first film 2a and/or the second film 2b, or a crease may be merely added. Alternatively, the first film 2a and the second film 2b may be distinguished from each other by the thicknesses thereof. In this case, for example, if the first film 2a and the second film 2b are formed from the same material, the thicker film has a slower degradation time than the thinner film, so that the thicker film is placed on the mucosal side (in the example in FIG. 1(C), the first film 2a is the thicker film and the second film 2b is the thinner film).

The above-described differences in appearance, shape, etc., do not need to be provided on the entire surface of the first film 2a and/or the second film 2b, it is sufficient if the surgeon can distinguish the first film 2a and the second film 2b from each other, and the differences can be provided at a desired location in the bag 1 (the first film 2a and/or the second film 2b).

The breaking strength of each of the first film 2a and the second film 2b can be 4N to 14N, and, from the viewpoint of peelability, etc., the breaking strength is preferably 6 N or more as a reference value for the strength for molding. This breaking strength is the maximum value of pressure when a film (15 mm × 60 mm × 0.05 mm) set in a tensile testing machine (Force Tester MCT-1150, manufactured by A&D Company, Limited) is pulled at a speed of 300 mm/min and either breaks or stretches to its maximum.

### (Opening 3)

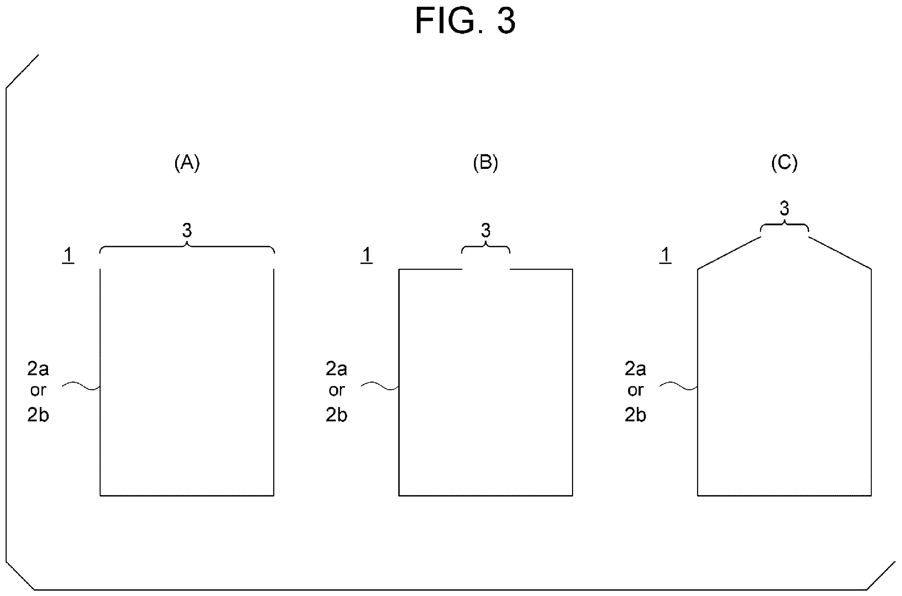

The bag 1 has an opening 3 between the first film 2a and the second film 2b which are superimposed and bonded with each other at edge portions thereof. That is, in the bag 1, the first film 2a and the second film 2b are superimposed and bonded (sealed) with each other, but in a part of the bag 1, the first film 2a and the second film 2b are not bonded with each other and the opening 3 is provided. For example, if the bag 1 has a quadrangular sheet shape, the bag 1 may be open in one direction out of the four directions (FIG. 2), or may be open in two directions. The bone substitute can be inserted through the opening 3 and contained in the bag 1. The width of the opening 3 may be the same as the length of one side of the bag 1 (the first film 2a and the second film 2b) as shown in FIG. 3(A), or may be smaller than the length of one side of the bag 1 as shown in FIG. 3(B). In addition, a side portion of the bag 1 in which the opening 3 is provided may have a shape that is tapered toward the outside of the bag 1 as shown in FIG. 3(C). If the bag 1 has such a tapered shape, as compared to the mode in FIG. 3(A), the bag 1 can be brought into the inside of the mouth through the gap between the teeth in front of and behind the tooth loss site without trimming the bag 1 in advance, and can be easily slipped into the gap between the alveolar bone and the gum at that site. In other words, workability during the procedure is improved. The width of the opening 3 can be set and changed as appropriate as long as the bone substitute can be inserted. After the bone substitute is contained, the opening 3 may be sealed (including heat-sealing and folding). In order to prevent the bone substitute from leaking out, it is preferable to heat-seal the opening 3 during the procedure.

The bone substitute to be contained in the bag 1 and used for bone augmentation is not particularly limited, and may be an autologous bone, an allogeneic bone, an artificial bone, or the like. The bone substitute is a distributed material, and bone substitutes from various companies can also be used.

### [Manufacturing method for bag 1]

The manufacturing method for the bag 1 is not limited, but the so-called flow casting method (solution casting method) can be used. That is, for example, a prepared PLGA solution is spread evenly over a smooth surface (substrate such as glass or a silicon wafer) (casting, flow casting). Then, the first film 2a and the second film 2b are formed by drying. Then, the first film 2a and the second film 2b are superimposed and sealed on each other (in the example shown in FIG. 2(A), sealing is performed in the three directions other than the opening 3). Thus, the bag 1 can be manufactured. In addition, the bag 1 of the present disclosure is not limited to a bag in which two films are superimposed on each other, and in the case where the bag 1 is manufactured using only one type of film (2a or 2b) (the in vivo degradation time of the bag 1 is the same for both surfaces), sealing may be performed in two directions. For example, if a film of 40 mm × 50 mm is prepared to manufacture a bag 1 of 40 mm × 25 mm, a long side (50 mm) portion may be folded in half, and only 25 mm portions at two opposing sides may be sealed (in the vertical direction in FIG. 2(B)), or a 40 mm portion and a 25 mm portion at two adjacent sides may be sealed (in the vertical and horizontal directions in FIG. 2(C)). Thus, in the manufacturing process for the bag 1 in which the first film 2a and the second film 2b are formed from the same one type of film, the number of times of sealing can be reduced by providing a folding step.

Here, the PLGA solution is prepared by dissolving PLGA in an appropriate organic solvent. Examples of the organic solvent include chloroform, dichloromethane, etc. This solvent is used for processing PLGA into a film shape. The PLGA concentration of the solution may differ for each film, and is adjusted as appropriate according to the desired film thickness and physical properties. In the above-described flow casting method, the viscosity of the PLGA solution is preferably 15 mPa.s or lower.

The prepared PLGA solution can be spread using a spin coater, or can also be spread manually using a pipette or spreader. Drying can be performed at room temperature until the solvent evaporates, or in an environment with a temperature controlled for acceleration. The time until complete drying varies depending on the concentration of the solution and environmental conditions.

In the case where the bag 1 (or one of the first film 2a and the second film 2b) is formed of a collagen membrane, the bag 1 can be manufactured by processing a commercially available material into the desired shape. Each film may be sewed with a thread, or may be bonded with each other using an adhesive or by heat treatment (heat-sealing). A collagen membrane and a PLGA membrane can be bonded with each other by heat treatment, or different types of collagen membranes can also be bonded with each other by heat treatment.

### [Application of bag 1]

As described above, the bag 1 is used for bone augmentation (bone grafting). As an example, a method of bone augmentation using the bag 1 can include a step of making an incision in the gum in the mouth, a step of placing the bag 1 containing the bone substitute on the missing part of the alveolar bone (see FIG. 1(C)), and a step of suturing the gum. The bone substitute contained in the bag 1 is in a bag-like shape and therefore does not move. Thus, even if the bone substitute is placed, it is possible to ensure that the bone substitute does not move from the site where the bone substitute is placed.

In addition, another method of bone augmentation using the bag 1 may include a step of making an incision in the gum in the mouth, a step of placing the bag 1 containing the bone substitute on the missing part of the alveolar bone so as to cover an implant when placing the implant in the missing part of the alveolar bone (in the state in FIG. 1(A)) (see FIG. 1(E) or (F)), and a step of suturing the gum. Finally, an artificial tooth (crown) is attached, and the treatment site of the patient is brought into a state in FIG. 1(D) (the bag 1 is decomposed and disappears).

Here, the "implant" in the step of placing the bag 1 containing the bone substitute on the missing part of the alveolar bone so as to cover the implant may be either a bone-level implant or a tissue-level implant, and may be not only an implant (fixture) but also an implant and a cover screw (so-called two-piece type. See FIG. 1(E)), an implant and a healing cap (see FIG. 1(F)), or an implant and a healing abutment. The procedure for implant placement may be placement by a one-stage method (FIG. 1(F)) or placement by a two-stage method (FIG. 1(E)), and the bag 1 can be used in a wide range of procedures for implant placement. As specific examples, in the case where a bone-level implant or a tissue-level implant is placed by the one-stage method, a healing abutment or a healing cap can also be used in combination, and in the case where a bone-level implant or a tissue-level implant is placed by the two-stage method, a cover screw can also be used in combination.

As another application of the bag 1 of the present disclosure, the bag 1 can be used as a material with which a tooth extraction socket is filled (recess after tooth extraction) when a tooth is extracted. That is, immediately after tooth extraction, resorption of the alveolar bone is suppressed by the bag 1 placed in the tooth extraction socket, so that, when filling with the bag 1, the bag 1 is more useful for increasing the bone volume when placing an implant in the future, than when not filling with the bag 1. With the bag 1 composed of a plurality of films, this suppression effect is significant, and the bone substitute is contained and used (simultaneous bone augmentation at the time of tooth extraction). In this application as well, the shape of the bag 1 is not particularly limited, and the bag 1 can be transformed (folded) as appropriate to match the tooth extraction socket. In addition, when the tooth extraction socket is filled with the bag 1 after tooth extraction, the use amount (required amount) of the bone substitute in implant treatment for that patient can be reduced.

### [Bag 1']

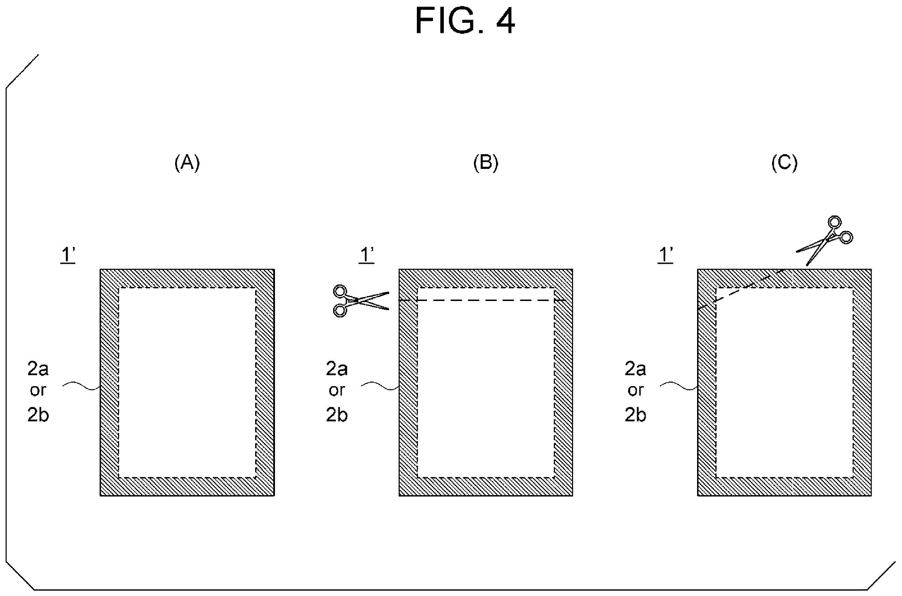

According to the present disclosure, a bag 1' which is different from the bag 1 described above in that the bag 1' does not have an opening 3, is provided (see FIG. 4(A)). That is, the edge portions of the first film 2a and the second film 2b are superimposed and bonded (sealed) with each other. The content of the bag 1' (the first film 2a and/or the second film 2b which are superimposed and bonded with each other) is not limited, and may or may not necessarily contain air (gas) or the like. The application of the bag 1' is the same as that of the bag 1, and the bag 1' can be used for bone augmentation, implant treatment, etc., but in these procedures, in order to cut the bag 1' at a desired location by the surgeon (FIGS. 4(B) and (C)) and contain the bone substitute in the bag 1' and/or in order to place the bag 1' at a treatment site, the bag 1' can be made into a desired shape to suit the case.

The manufacturing method for the bag 1' is also the same as the manufacturing method for the bag 1, but, since the bag 1' does not have an opening 3 at the manufacturing stage as described above, the bag 1' is sealed at four sides (all edges) thereof. As described above, this sealing method includes sewing with a thread, heat-sealing, etc., and may further include interfolding in the case where the first film 2a and the second film 2b are formed from the same one type of film.

In the bag 1 according to the present disclosure configured as described above and the manufacturing method for the bag 1, the bag 1 is used for bone augmentation, includes the first film 2a and the second film 2b each made of a biodegradable component, and has the opening 3 between the first film 2a and the second film 2b superimposed and bonded with each other. The in vivo degradation time of the first film 2a is equal to or longer than the in vivo degradation time of the second film 2b, and if the in vivo degradation times of the first film 2a and the second film 2b are different from each other, the first film 2a and the second film 2b are different from each other so as to be visually or tactilely identifiable. Furthermore, the bag 1 may contain the bone substitute.

The bag 1 according to the present disclosure and the bag 1 obtained by the manufacturing method according to the present disclosure have a bag shape that allows the bone substitute to be contained and held through at least the one opening 3. Thus, in a procedure for bone formation, the contained bone substitute can be fixed by merely placing the bag 1 at the missing part of the alveolar bone, so that user-friendly bone augmentation work that does not require complicated operations as in Technique III to V can be realized. Furthermore, the bag 1 composed of the first film 2a and the second film 2b each made of a biodegradable component is a novel fixation means different from the single resorbable barrier membranes used in Techniques II to IV and the titanium frame and the non-resorbable membrane used in Technique V. In addition, in the bag 1, the first film 2a and the second film 2b having different in vivo degradation times (bioresorbable performances) can also be used, and it is possible to design the bag 1 according to the treatment site (mucosal side, bone side).

In addition, in the bag 1 according to the present disclosure and the manufacturing method for the bag 1, the biodegradable component may include collagen. Thus, the material that has been conventionally recognized as safe for use on the human body can also be used in the user-friendly bone augmentation work according to the present disclosure.

In addition, in the bag 1 according to the present disclosure and the manufacturing method for the bag 1, the biodegradable component may include a lactic acid (L)-glycolic acid (G) copolymer. Thus, it is preferable to use PLGA for the first film 2a and the second film 2b as described above, since, for example, PLGA is degraded more quickly than when polylactic acid is used alone. As the biodegradable component, it is also possible to use bovine collagen, atelocollagen, tendon collagen, porcine collagen, a placenta membrane, lactic acid, or glycolic acid. As a combination of biodegradable components forming the first film 2a and the second film 2b, one film may be formed from one of these materials, and the other film may be formed from PLGA.

In addition, in the bag 1 according to the present disclosure and the manufacturing method for the bag 1, the UG ratio of each of the first film 2a and the second film 2b is in the range of 45/55 to 88/12, and the proportion of lactic acid in the UG ratio of the first film 2a may be equal to or higher than the proportion of lactic acid in the UG ratio of the second film 2b. Moreover, the UG ratio of the first film 2a may be in the range of 55/45 to 88/12, and the UG ratio of the second film 2b may be in the range of 45/55 to 55/45. As described above, the in vivo degradation time (bioresorbable performance) of each film can be adjusted.

In addition, in the bag 1 according to the present disclosure and the manufacturing method for the bag 1, the inherent viscosity of the lactic acid-glycolic acid copolymer may be in the range of 0.6 dL/g to 1.4 dL/g. In terms of required strength and hardness of the bag 1, it is preferable that the inherent viscosity of PLGA is in this range.

In addition, in the bag 1 according to the present disclosure and the manufacturing method for the bag 1, each of the first film 2a and the second film 2b may be formed by spreading a material containing the biodegradable component on a flat surface and then drying the material. Thus, the bag 1 can be easily manufactured.

In the bag 1' according to the present disclosure and the manufacturing method therefor, the bag 1' is used for bone augmentation, and includes the first film 2a and the second film 2b each made of a biodegradable component. The edge portions of the first film 2a and the second film 2b are bonded with each other, the in vivo degradation time of the first film 2a is equal to or longer than the in vivo degradation time of the second film 2b, and if the in vivo degradation times of the first film 2a and the second film 2b are different from each other, the first film 2a and the second film 2b are different from each other so as to be visually or tactilely identifiable. Since the edge portions (four sides) of the bag 1' are sealed as described above, the surgeon can process the bag 1' into a desired shape according to the procedure.

The bags 1 and 1' according to the present disclosure and the manufacturing method for the bag 1 are not limited to the above-described modes and combinations, and various modifications can be made.

For example, in addition to the first film 2a and the second film 2b, the bag 1 or 1' may include another bioresorbable film, and the material of the bioresorbable film may be the same as or different from those of the first film 2a and the second film 2b. For example, it is also possible to provide multiple types of resorbable films on one surface of the bag 1.

In addition, the shapes of the bags 1 and 1' may be formed into various shapes such as a columnar shape, a conical shape, a spherical shape, a rectangular parallelepiped shape, and a tooth shape in accordance with the row of teeth and the missing part of the alveolar bone, in addition to a sheet shape. Each surface of the bag 1 or 1' may have a laminated structure.

In the bag 1, the position of the opening 3 is not limited to the position between the first film 2a and the second film 2b, and the first film 2a and the second film 2b may be superimposed and bonded with each other at the edge portions thereof (closed in the four directions of the bag 1), and the opening 3 may be formed in the first film 2a or the second film 2b. The first film 2a and the second film 2b may be made different visually or tactilely from each other by the opening 3. The number of openings 3 is also not limited to one. A plurality of openings 3 may be provided between or in the first film 2a and the second film 2b which are superimposed and bonded with each other.

### EXAM PLES

Hereinafter, the present disclosure will be described in more detail by means of examples (experimental examples). Specifically, bags 1 as shown in FIG. 3(A) were manufactured using the following materials, and the degradability (i.e., in vivo degradation time and bioresorbable performance) of each bag 1 (example) was examined. The first film 2a and the second film 2b were formed with the same composition, and thus are referred to simply as film or membrane in the following description.

### (1) Experimental reagents

### · PLGA solution:

PLGA:
DLG 8513 E Lot No. 0002633992, manufactured by Ashland
DLG 8509 A Lot No. 0002480336, manufactured by Ashland
DLG 8507 A Lot No. 0002591847, manufactured by Ashland
DLG 7507 A Lot No. 0002510702, manufactured by Ashland
PLGA 5-50, manufactured by Mitsui Chemicals, Inc.

Dichloromethane (solvent): Lot No. DLR5072, manufactured by FUJIFILM Wako Pure

### Chemical Corporation

### · Additives

Tween 80: Lot No. 104K1486, manufactured by KANTO CHEMICAL CO.,INC.
Food Yellow No. 5: Lot No. R10401M1, manufactured by Osaka Shokuhin Shikiso K.K. (used in Example 5 as a coloring agent for type identification)
Riboflavin: Lot No. CJCTO-YT, manufactured by Tokyo Chemical Industry Co., Ltd. (used in Example 6 as a coloring agent for type identification)

### · Elution solution:

Polyvinyl alcohol EG-05P: Lot No. 68N69, manufactured by The Nippon Synthetic Chemical Industry Co., Ltd.
Tween 80: Lot No. 104K1486, manufactured by KANTO CHEMICAL CO.,INC.
Lactic acid: Lot No. 010B2087, manufactured by KANTO CHEMICAL CO.,INC.
Distilled water: Lot No. 18D671 and Lot No. 18D685, manufactured by Kyoei Pharmaceutical Co., Ltd.

### · Rinse water:

Distilled water: Lot No. 18D671 and Lot No. 18D685, manufactured by Kyoei Pharmaceutical Co., Ltd.

### (2) Experimental apparatus

Micrometer: M110-25, manufactured by Mitutoyo Corporation
Sealer: POLYSEALER P-200, manufactured by FUJI IMPULSE CO.,LTD.
Tabletop tensile and compression testing machine: Force Tester MCT-1150, manufactured by A&D Company

### (3) Experimental method

### · Bag production:

Specified amounts of PLGA and Tween 80 were weighed, each dissolved in dichloromethane in a sealed container, and left overnight. The volume of each solution was set to 25 mL (32 g), the concentration of the PLGA solution was set to 2.5% w/w (0.8 g/32 g), and the additive amount was set to 0.5% w/w (0.16 g/32 g, 20% relative to PLGA). The resulting solution was poured into a stainless steel tray by flow casting. The size of the stainless steel tray was 105 × 135 mm for a film thickness of 0.05 mm and 130 × 195 mm for a film thickness of 0.03 mm. The solution was left (naturally dried) at least overnight, each film was then peeled off, the state thereof was visually inspected, and the film thickness was measured.

For each example, a bag 1 (25 mm × 40 mm) and a test piece (15 mm × 60 mm) were produced from the peeled film. The breaking strength was measured using the test piece (tensile speed: 300 mm/min).

### · Degradation time confirmation (elution test):

One bag 1 was precisely weighed into a 125 mL glass container, 100 mL of an elution solution (2% PVA aqueous solution : 20% Tween 80 aqueous solution : 0.9% lactic acid aqueous solution / water = 40:1:40/200) heated at 37±0.5°C was added, and a cap equipped with a septum having a thickness of 2 mm was put on the container. Each container was fixed and immersed in a water bath at 37±0.5°C. During the elution test, the temperature of the water bath was increased from 37°C to 61°C at a rate of 0.5°C/hour. After 3 hours (38.5°C), 28 hours (51.0°C), and 48 hours (61.0°C) from the start of temperature increase, each container was taken out, and the solution was filtered. The taken-out film was washed with water and then dried. The weight of the film was measured to check the weight maintenance rate thereof.

In Examples 1 to 4, the above method was repeated four times, sampling at each time (after 3, 28, and 48 hours) was assumed to be 1 to 12 months after placement in a body, and the samples were checked at 9 points (1, 3, 4, 6, 7, 8, 9, 10, and 12 months).

However, in Examples 5 and 6, the above method was repeated twice, sampling at each time was assumed to be 1 to 6 months after placement in a body, and the samples were checked at 6 points (1, 2, 3, 4, 5, and 6 months).

### (4) Results of degradation confirmation test

### [Example 1: DLG 8513 E film (0.05 mm) (L/G ratio: nominal value 85/15, specification 82.0/18.0 to 88.0/12.0, inspection value 84.5/15.5 (measured by ¹H-NMR spectroscopy in accordance with USP<761>), IV: 1.30 dL/g, breaking strength: 13.62 N)]

The weight maintenance rate decreased at the third month, and then remained almost unchanged. The film curled in appearance, and at the ninth month, the film became transparent when dried, and became brittle.

### [Example 2: DLG8509 A film (0.05 mm) (L/G ratio: nominal value 85/15, specification 82.0/18.0 to 88.0/12.0, inspection value 84.5/15.5 (measured by ¹H-NMR spectroscopy in accordance with USP<761>), IV: 0.99 dL/g, breaking strength: 12.72 N)]

The weight maintenance rate decreased at the third month, and then remained almost unchanged. The moisture taken in at the sixth and eighth months was difficult to dry and exceeded the previous month. At the sixth month, changes in appearance began to be seen before and after drying, and the film became brittle when dried, and was easily cracked.

### [Example 3: DLG 8507 A film (0.05 mm) (L/G ratio: nominal value 85/15, specification 82.0/18.0 to 88.0/12.0, inspection value 84.6/15.4 (measured by ¹H-NMR spectroscopy in accordance with USP<761>), IV: 0.64 dL/g, breaking strength: 8.36 N)]

The weight maintenance rate decreased significantly at the third month, and then gradually decreased. The film became transparent in appearance after drying at the fourth month, became brittle when dried at the sixth month, and was brittle even before drying at the seventh month.

### [Example 4: DLG 7507 A film (0.05 mm) (L/G ratio: nominal value 75/25, specification 72.0/28.0 to 78.0/22.0, inspection value 74.9/25.1 (measured by ¹H-NMR spectroscopy in accordance with USP<761>), IV: 0.68 dL/g, breaking strength: 8.38 N)]

The weight maintenance rate decreased significantly at the third month, and then gradually decreased. The film became transparent in appearance after drying at the fourth month, became brittle when dried at the fourth month, and was brittle even before drying at the seventh month.

### [Example 5: PLGA 5-50 colored film (0.05 mm) (L/G ratio: nominal value 50/50, specification 45.0/55.0 to 55.0/45.0, inspection value 50.5/49.5 (measured by ¹H-NMR spectroscopy), IV: 0.50 dL/g, breaking strength: 4.78 N)]

The weight maintenance rate decreased in the second to third months, and remained almost unchanged in the third to fifth months. The weight maintenance rate began to decrease again in the fifth to sixth months. The film curled in appearance until the second month, and when dried, the film became transparent but maintained its shape. At the third month, the film became brittle when dried. Even when a dye was mixed in film production, no difference in physical properties was recognized.

### [Example 6: DLG 7507 A colored film (0.03 mm) (breaking strength: 7.22 N)]

The weight maintenance rate decreased significantly at the second month, and then gradually decreased. The film began to curl in appearance from the second month, and when dried, the film became transparent but maintained its shape. At the sixth month, the film became quite brittle. Compared to the film having a film thickness of 0.05 mm (Example 4), no significant difference in weight maintenance rate and appearance was confirmed, so that it was found that if the film thickness is in the range of 0.03 to 0.05 mm, it is possible to produce the bag 1 without any problem with strength.

### (5) Summary

Regarding the maintenance period, no significant changes (differences) were seen in weight, and based on appearance and brittleness, the following tendencies were found in relation to the UG ratio.

If the UG ratio is 85/15, the film becomes brittle at 6 months or longer.

If the UG ratio is 75/25, the film becomes brittle at 4 months or longer.

If the UG ratio is 50/50, the film becomes brittle at 4 months or shorter.

From the above findings, it can be understood that if the UG ratios of the first film 2a and the second film 2b constituting the bag 1 are varied, the first film 2a and the second film 2b have different degradability (in vivo degradation time and bioresorbable performance). In addition, it is suggested that if the UG ratio of the first film 2a is in the range of 55/45 to 88/12 and the L/G ratio of the second film 2b is 45/55 to 55/45, the in vivo degradation times of the first film 2a and the second film 2b are about 4 to 9 months and 1 to 4 months, respectively.

## Claims

1. A bag used for bone augmentation, the bag comprising
a first film and a second film each made of a biodegradable component, wherein
the bag has an opening between the first film and the second film superimposed and bonded with each other,
an in vivo degradation time of the first film is equal to or longer than an in vivo degradation time of the second film, and
if the in vivo degradation times of the first film and the second film are different from each other, the first film and the second film are different from each other so as to be visually or tactilely identifiable.

2. The bag according to claim 1, wherein the biodegradable component includes collagen.

3. The bag according to claim 1, wherein the biodegradable component includes a lactic acid (L)-glycolic acid (G) copolymer.

4. The bag according to claim 3, wherein
an UG ratio of each of the first film and the second film is in a range of 45/55 to 88/12, and
a proportion of lactic acid in the UG ratio of the first film is equal to or higher than a proportion of lactic acid in the UG ratio of the second film.

5. The bag according to claim 3 or 4, wherein an L/G ratio of the first film is in a range of 55/45 to 88/12.

6. The bag according to any one of claims 3 to 5, wherein an L/G ratio of the second film is in a range of 45/55 to 55/45.

7. The bag according to any one of claims 3 to 6, wherein an inherent viscosity of the lactic acid-glycolic acid copolymer is in a range of 0.6 dL/g to 1.4 dL/g.

8. The bag according to any one of claims 1 to 7, wherein the bag contains a bone substitute.

9. A method for manufacturing the bag according to claim 1, the method comprising
forming each of the first film and the second film by spreading a material containing the biodegradable component over a flat surface and then drying the material.

10. A bag used for bone augmentation, the bag comprising
a first film and a second film each made of a biodegradable component, wherein
edge portions of the first film and the second film are bonded with each other,
an in vivo degradation time of the first film is equal to or longer than an in vivo degradation time of the second film, and
if the in vivo degradation times of the first film and the second film are different from each other, the first film and the second film are different from each other so as to be visually or tactilely identifiable.
